# FASCICULE DE BREVET EUROPEEN

(11) **EP 2 304 050 B1**
(45) Date de publication et mention de la délivrance du brevet: **21.08.2013**
(21) Numéro de dépôt: 09797511.4
(22) Date de dépôt: 16.07.2009
(51) Int. Cl.: C12Q 1/56, G01N 33/86

(54) **MÉTHODE DE DOSAGE IN VITRO DU FACTEUR TISSULAIRE CIRCULANT - APPLICATION À LA DÉTECTION DES DÉSORDRES DE LA COAGULATION**
VERFAHREN FÜR EIN IN-VITRO-ASSAY DES ZIRKULIERENDEN THROMBOPLASTINS UND SEINE VERWENDUNG BEI DER ERKENNUNG VON BLUTGERINNUNGSERKRANKUNGEN
METHOD FOR IN VITRO ASSAY OF THE CIRCULATING TISSUE FACTOR, AND USE IN THE DETECTION OF COAGULATION DISORDERS

(30) Priorité: 17.07.2008 FR 0804073
(43) Date de publication de la demande: 06.04.2011
(73) Titulaire: Diagnostica Stago, 92600 Asnières (FR)
(72) Inventeur: VAN DREDEN, Patrick, F-29690 Brennilis (FR); ROUSSEAU, Aurélie, F-78430 Louveciennes (FR); WOODHAMS, Barry, F-95130 Le Plessis Bouchard (FR)
(74) Mandataire: Desaix, Anne
(86) Numéro de dépôt international: PCT/EP2009/059182
(87) Numéro de publication internationale: WO 2010/007140

(56) Documents cités:
- EP-A- 1 367 135
- WO-A-2006/096345
- US-A- 5 705 396
- BENDZ B ET AL: "A new sensitive chromogenic substrate assay of tissue factor pathway inhibitor type 1." THROMBOSIS RESEARCH 15 MAR 2000, vol. 97, no. 6, 15 mars 2000 (2000-03-15), pages 463-472, XP002505261 ISSN: 0049-3848
- FUKUDA C ET AL: "Measuring tissue factor (factor III) activity in plasma." CLINICAL CHEMISTRY SEP 1989, vol. 35, no. 9, septembre 1989 (1989-09), pages 1897-1900, XP002505262 ISSN: 0009-9147
- HISCHE E A ET AL: "Spectrophotometry of tissue thromboplastin in cerebrospinal fluid." CLINICAL CHEMISTRY AUG 1981, vol. 27, no. 8, août 1981 (1981-08), pages 1427-1430, XP002505263 ISSN: 0009-9147
- BLADBJERG E M ET AL: "In vitro effects of heparin and tissue factor pathway inhibitor on factor VII assays. possible implications for measurements in vivo after heparin therapy" BLOOD COAGULATION & FIBRINOLYSIS, RAPID COMMUNICATIONS, OXFORD,OXFORD, GB, vol. 11, no. 8, 1 décembre 2000 (2000-12-01), pages 739-745, XP009069876 ISSN: 0957-5235
- DATABASE BIOSIS BIOSCIENCES INFORMATION SERVICE, PHILADELPHIA, PA, US; novembre 2004 (2004-11), SMITH STEPHANIE A ET AL: "Do elevated plasma tissue factor pathway inhibitor (TFPI) levels affect measurement of factor VIIa?" XP002505264 & BLOOD, vol. 104, no. 11, Part 1, novembre 2004 (2004-11), page 537A, 46TH ANNUAL MEETING OF THE AMERICAN-SOCIETY-OF-HEMATOLOGY; SAN DIEGO, CA, USA; DECEMBER 04 -07, 2004
- DATABASE MEDLINE US NATIONAL LIBRARY OF MEDICINE (NLM), BETHESDA, MD, US; février 1995 (1995-02), MURAKAMI F ET AL: "[An improved assay for plasma tissue factor activity]" XP002505265 & RINSHO BYORI. THE JAPANESE JOURNAL OF CLINICAL PATHOLOGY FEB 1995, vol. 43, no. 2, février 1995 (1995-02), pages 137-141,
- KRUPINSKI JERZY ET AL: "Blood-borne tissue factor activity predicts major cerebrovascular events in patients undergoing carotid endarterectomy: results from a 1-year follow-up study." CEREBROVASCULAR DISEASES (BASEL, SWITZERLAND) 2008, vol. 25, no. 1-2, 22 novembre 2007 (2007-11-22), pages 32-39, XP008098991 ISSN: 1421-9786
- EILERTSEN K-E ET AL: "TISSUE FACTOR: (PATHO)PHYSIOLOGY AND CELLULAR BIOLOGY" BLOOD COAGULATION & FIBRINOLYSIS, RAPID COMMUNICATIONS, OXFORD,OXFORD, GB, vol. 15, no. 7, 1 janvier 2004 (2004-01-01), pages 521-538, XP008065964 ISSN: 0957-5235

## Description

L'invention concerne le domaine de l'hémostase, et en particulier les désordres de la coagulation sanguine liés à une expression anormale du facteur tissulaire, ainsi que les phénomènes physiopathologiques corrélés à une surexpression de ce facteur.

La présente invention a pour objet une méthode de dosage de l'activité du facteur tissulaire circulant dans un échantillon biologique telle que définie dans les revendications.

La méthode de l'invention est réalisée *in vitro,* en particulier sur un échantillon de sang prélevé chez un patient, ou sur échantillon préparé à partir d'un échantillon de sang prélevé.

La méthode de l'invention est préférentiellement réalisée en milieu plasmatique. Avantageusement, elle met en oeuvre un dosage de type chromogénique.

Le facteur tissulaire, principal activateur de la coagulation *in vivo*, est une glycoprotéine transmembranaire de 47 kDa dont la liaison avec le facteur VII ou VIIa permet la formation du complexe facteur tissulaire-facteur VIIa qui, par l'activation des facteurs IX et X, déclenche l'activation de la coagulation, entrainant la génération de thrombine et la formation de fibrine. En effet l'initiation de la coagulation nécessite la liaison du facteur VII à son récepteur protéique spécifique de surface, le facteur tissulaire, exprimé par un certain nombre de cellules ou par leurs composants (endothélium, monocytes-macrophages, cellules musculaires lisses, fibroblastes, épithéliums, microparticules d'origine cellulaire, plaquettes après transfert moléculaire à partir de microparticules d'origine monocytaire, cellules néoplasiques). L'activité du complexe facteur tissulaire-VIIa est impliquée non seulement dans l'hémostase normale mais également dans la survenue de thromboses associées aux maladies malignes, aux syndromes coronaires aigus et au sepsis sévères. La régulation de cette activité essentielle au maintien de l'équilibre hémostatique est assurée par un inhibiteur de la voie du facteur tissulaire, le TFPI (tissue factor patwhay inhibitor). Cet inhibiteur, le TFPI inactive non pas le facteur tissulaire mais l'activité catalytique du complexe facteur tissulaire-facteurVIIa. Dans un premier temps le TFPI se lie au facteur Xa, le facteur Xa est alors inhibé ; dans un second temps, le complexe Xa/TFPI va se lier au complexe facteurVIIa-tissue facteur pour former un complexe quaternaire facteurXa/TFPI/facteurVIIa/facteur tissulaire, au sein duquel le facteur Xa, le facteur VIIa et le facteur tissulaire n'ont plus d'activité. Le TFPI limite donc l'activation de la coagulation induite par l'expression du Facteur Tissulaire (FT), imposant une sorte de palier initial à gravir avant que les évènements moléculaires initiaux de la coagulation n'induisent réellement une activation de la cascade de la coagulation. Le TFPI permet donc une exposition de Facteur Tissulaire à bas bruit, sans conséquence procoagulante. Le TFPI est synthétisé par les cellules endothéliales et par les plaquettes.

Dans les conditions physiologiques, l'expression du facteur tissulaire est ubiquitaire et il est présent à la surface de types cellulaires très variés, à l'exception des cellules au contact direct de la circulation sanguine (cellules endothéliales et éléments figurés du sang). L'adventice des vaisseaux, le myocarde, les tissus muqueux et épidermiques sont très riches en facteur tissulaire.

Les thromboses sur plaques d'athérosclérose, responsables d'accidents artériels ischémiques aiguës, sont en grande partie liées à l'exposition pathologique de facteur tissulaire. La thrombogénicité des plaques athéroscléreuses est corrélée à la quantité de facteur tissulaire présent, celui-ci renforçant considérablement le processus de thrombogénicité en cas de rupture des plaques. De même, au cours du sepsis, le facteur tissulaire peut être exprimé par les monocytes et les cellules endothéliales et être responsable d'une coagulation intravasculaire disséminée. Le facteur tissulaire a aussi été identifié à la surface de micro-particules issues de membranes cellulaires. Ces microparticules, provenant de monocytes et lymphocytes ont été identifiées dans les plaques d'athérosclérose. Elles possèdent une activité procoagulante et sont issues de cellules apoptotiques.

Outre son activité procoagulante, le facteur tissulaire est aujourd'hui considéré comme une protéine ayant des fonctions comparables à celles d'une hormone sur différentes populations cellulaires (1).

Ainsi, par exemple, il a été montré que le facteur tissulaire augmente la production de facteur de croissance endothélial vasculaire (VEGF) et induit une angiogénèse *in vivo* et *in vitro.*

Le facteur tissulaire stimule également la migration cellulaire, et son implication dans des métastases et dans l'organisation et l'intégrité de la paroi des vaisseaux dans l'angiogénèse associée à des tumeurs a déjà été décrite (2).

Il est donc clair aujourd'hui que le facteur tissulaire intervient dans de nombreuses situations physiopathologiques, et qu'il est de ce fait nécessaire de disposer d'une méthode de dosage permettant de suivre les variations de son activité non seulement dans des situations de risques thrombotiques mais également dans le suivi de l'évolution de certaines pathologies, notamment cancéreuses.

Les méthodes de dosage du facteur tissulaire sanguin ou plasmatique sont classiquement les méthodes de type immunologique, telles que les méthodes de type ELISA ne mesurant que l'antigène et donc des dosages quantitatifs et non qualitatifs.

Différents tests d'activité ont été illustrés dans la littérature, la plupart étant réalisés à partir d'extraits cellulaires par méthode chronométrique (mesure du temps de formation d'un caillot) selon le principe décrit dans (3).

D'autres tests d'activité du TF utilisés consistent à évaluer la génération de facteur X activé (FXa) en présence de facteur VII activé (FVIIa) et de calcium et d'un substrat spécifique du facteur Xa.

Le test consiste alors à mesurer la capacité du complexe TF-FVIIa à activer le FX en FXa.

Cette activation du FX peut être calculée à partir de la quantité de substrat hydrolysé, par exemple par une méthode chromogénique, lorsqu'on utilise un substrat dont l'hydrolyse génère un groupement chromogène.

La plupart des tests d'activité basés sur ce schéma se font sur des échantillons de plasma préalablement défibrinés, c'est-à-dire traités de façon à éviter une polymérisation des monomères de fibrine qui pertuberait la lecture optique lors de la réalisation des essais. Cette étape préalable complique cependant l'automatisation de ce type de test et nécessite d'effectuer le test en deux temps.

Il est ainsi par exemple décrit par Fukuda et al. (Measuring Tissue Factor (Factor III) Activity in Plasma, Clin. Chem. 35/9, 1897-1900 (1989)) un dosage du facteur tissulaire dans le plasma, à partir d'un échantillon prétraité pour en enlever la fraction euglobuline, l'échantillon ayant également été prétraité par chauffage pour en enlever le fibrinogène et donc pour éviter la formation de fibrine.

Dans le cadre de mesures particulières de paramètres de l'hémostase, tel que relevé par EP 1 367 135, il est également connu l'emploi de substances qui inhibent la polymérisation de la fibrine, cette inhibition pouvant néanmoins rester incomplète.

Par ailleurs, les tests d'activité de l'état de l'art ne tiennent pas compte des variations de taux de TFPI dans le plasma, qui peuvent fausser l'activité réelle de TF mesurée, et donc donner des informations incorrectes sur le potentiel thrombogène d'un malade lié à son taux de TF.Par ailleurs, si l'état de l'art, tel que décrit par exemple dans la demande WO 2006/096345, mentionne l'emploi d'inhibiteurs de TFPI dans le cadre de tests associés à la coagulation dans des échantillons de plasma, il ne donne aucune indication sur l'emploi de tels agents dans le cadre de la détection de l'activité du facteur tissulaire circulant.

Enfin, il est connu de Bendz et al. (A New Sensistive Chromogenic Substrate Assay of Tissue Factor Pathway Inhibitor Type 1, Thrombosis Research 97 (2000) 463-472.) un test de détection de l'activité de l'inhibiteur du TFPI dans un échantillon de plasma, sans lien avec la mesure de l'activité du facteur tissulaire circulant.

La présente invention vise donc à s'écarter des problèmes rencontrés dans les tests de l'état de l'art.

Pour ce faire, elle propose une méthode de dosage en un temps de l'activité du facteur tissulaire circulant, dans laquelle on neutralise l'effet inhibiteur du TFPI sur le facteur tissulaire.

L'invention consiste en effet à réaliser un dosage *in vitro* de l'activité du facteur tissulaire circulant sur un échantillon biologique en présence d'un inhibiteur de la polymérisation de la fibrine et d'un composé inhibant l'action du TFPI sur le facteur tissulaire tel que defini dans les revendications.

Plus précisément, la méthode selon l'invention permet de mesurer le facteur Xa généré par l'activité du facteur tissulaire circulant lorsqu'on ajoute au milieu réactionnel formé par l'échantillon biologique testé, l'inhibiteur de la polymérisation de la fibrine et l'inhibiteur de l'action du TFPI sur le FT, un excès des facteurs VII et X de la coagulation.

La présente invention a ainsi pour objet, une méthode de dosage *in vitro* en un temps dans un échantillon biologique de l'activité du facteur tissulaire circulant, ladite méthode étant basée sur la capacité du facteur tissulaire circulant à générer du facteur Xa en présence d'un excès de facteurs VII et X telle que definie dans les revendications.

L'invention consiste en une méthode de dosage *in vitro* de l'activité du facteur tissulaire circulant dans un échantillon biologique dérivé d'un prélèvement préalablement fait chez un patient dans laquelle on mesure la génération de facteur X activé dans un milieu réactionnel comprenant l'échantillon biologique testé, un excès de facteur VII et de facteur X, des ions calcium, un inhibiteur de la polymérisation de la fibrine et un inhibiteur de l'activité du TFPI.

L'échantillon biologique est préférentiellement un échantillon sanguin, et plus préférentiellement un échantillon de plasma.

La méthode de l'invention peut toutefois être également mise en oeuvre sur un échantillon d'urine ou de liquide cephalo-rachidien (LCR), ou autres prélèvements biologiques.

La méthode de l'invention est préférentiellement une méthode de type enzymatique, dans laquelle la quantité de Xa formée est déterminée par la mesure de l'activité d'hydrolyse de ce facteur sur un substrat spécifique.

Ce substrat est en particulier un substrat enzymatique, naturel ou synthétique. Il s'agit préférentiellement d'un substrat permettant de doser l'activité amidolytique du facteur Xa. En particulier, il s'agit d'un substrat chromogène ou fluorogène.

Un tel substrat, de type chromogène, est par exemple le CBS 5244 de la société Diagnostica Stago *(autres fournisseurs possibles: American Diagnostica; Biopep SA; Kabi Vitrum; Biogenic; Biophen; Chromogenix).*

La quantité de facteur Xa généré (et donc la mesure de l'activité du facteur tissulaire circulant) est calculée à partir du dosage de la quantité de substrat hydrolysé.

Ce dosage peut être réalisé en mesurant la variation de densité optique du mélange réactionnel dans une fenêtre de lecture déterminée en fonction du substrat choisi.

Dans un mode de réalisation particulier de l'invention, le plasma obtenu à partir de l'échantillon biologique prélevé chez le patient est dilué dans un tampon approprié.

II peut s'agir par exemple d'un tampon Owren Koller, fréquemment utilisé dans les tests d'hémostase.

La dilution réalisée dépend de la nature de l'échantillon testé et de la sensibilité du substrat utilisé.

Dans le cas d'un échantillon plasmatique, elle est avantageusement comprise entre 1/2 et 1/20, et préférentiellement comprise entre 1/2 et 1/4.

Les ions calcium sont par exemple apportés sous forme de CaCl₂.

L'inhibiteur de la polymérisation de la fibrine est choisi parmi les composés couramment utilisés dans l'état de l'art.

Il s'agit en particulier d'un inhibiteur polypeptidique, tel que l'oligopeptide GPRP.AcOH, dénommé commercialement Pefabloc et fourni par la société Pentapharm.

Cet inhibiteur peut être utilisé dans le milieu réactionnel à une concentration variant de 5 à 25 g/l, choisie selon la dilution du plasma testé. Par exemple, lorsque le plasma est dilué au 1/3, cet inhibiteur est avantageusement utilisé à une concentration de 10 g/l soit une *concentration finale* dans un intervalle de 0.666g/l à 3.33g/l), en particulier *de 1.332g*/l.

Bien que le Pefabloc constitue un inhibiteur de la polymérisation de la fibrine préféré pour la méthode de l'invention, il est également possible d'utiliser des inhibiteurs d'un autre type, tels que par exemple des anticorps anti-fibrinogène.

L'inhibiteur du TFPI est un composé qui inhibe les effets inhibiteurs du TFPI sur le TF circulant, permettant ainsi un dosage quantitatif du TF qui ne soit pas faussé par des variations du taux de TFPI présent dans l'échantillon.

L'inhibiteur du TFPI est choisi parmi tout type de composé à effet antagoniste du TFPI. Il s'agit préférentiellement d'anticorps ou de fragments d'anticorps anti-TFPI liant le TFPI, ces anticorps pouvant être monoclonaux, polyclonaux ou à simple chaine.

Selon un mode de réalisation particulier de l'invention, on utilise l'anticorps T4E2 qui est un anticorps monoclonal fourni par Diagnostica Stago.

Sa concentration est choisie de manière à inhiber tout le TFPI contenu dans l'échantillon testé. Elle peut varier en fonction de l'échantillon étudié, de 5 à 500 µg/ml (concentration dans le tampon de dilution). Dans le cas d'un échantillon de plasma dilué au 1/3, la concentration finale de l'anticorps est préférentiellement à 6,6 µg/ml.

Selon la méthode de l'invention, le milieu réactionnel comprend avantageusement un excès de facteurs VII et X. Ces facteurs permettent de mesurer l'activité du facteur tissulaire, puisque, comme cela l'a été précédemment expliqué, le facteur tissulaire se lie au facteur VII activé (FVIIa) pour former un complexe qui va alors activer les facteurs IX et X. Dans la mesure où l'activité du TF est mesurée via la quantité de facteur Xa produit, grâce à la mesure de l'hydrolyse d'un substrat spécifique du Xa, il est nécessaire que les facteurs VII et X ne soient pas limitants dans la réaction, afin que la quantité de Xa produit soit directement corrélée à l'activité du TF.

Ces facteurs sont donc ajoutés en excès, ce qui permet d'éviter un risque d'interférence de ceux-ci dans la mesure.

Selon le type d'échantillon testé, les concentrations finales de chacun de ces deux facteurs sont avantageusement comprises entre 5 et 20 PEU/mg/ml pour le facteur VII et entre 3 et 16 PEU/mg/ml pour le facteur X.

Selon un mode de réalisation particulier de l'invention, le facteur VII est à une concentration de 137 PEU/mg/ml et le facteur X à une concentration de 80 PEU/mg/ml lorsque l'échantillon est un plasma dilué au 1/3. (PEU : Plasma Equivalent Unit).

Ces facteurs peuvent être purifiés ou recombinants d'origine humaine ou animale. Ils sont par exemple commercialisés par par Dignostica Stago, Enzyme Research laboratories, Sigma.

Selon une variante préférée, l'échantillon de plasma testé contient en outre un composé inhibiteur de l'héparine, de façon à rendre le test indépendant de l'héparine éventuellement présente dans le plasma, par exemple dans le cadre du dosage d'un échantillon provenant d'un patient sous héparine.

Ce composé est par exemple le polybrène, classiquement utilisé pour inhiber les effets de l'héparine dans les tests d'hémostase.

Il est avantageusement utilisé dans une gamme de concentrations finales comprises entre 0,5 et 10 mg/l.

L'homme du métier est en mesure d'adapter les concentrations et activités, en fonction des réactifs choisis.

A titre illustratif, on notera que les concentrations ou activités des réactifs peuvent varier à hauteur de plus ou moins 50 %, par exemple à hauteur de plus ou moins 20 %, ou de plus ou moins 10 %, par rapport aux indications contenues dans la présente demande. Cette variation peut concerner chaque réactif, indépendamment des autres réactifs.

Selon un mode de réalisation particulier, la méthode de l'invention est réalisée suivant le protocole ci-après :

La mesure est réalisée sur un automate de la gamme STA commercialisé par la société Diagnostica Stago.

Un échantillon de plasma est dilué au 1/3 dans un tampon de dilution, composé d'un tampon Owren Koller, aditionné de polybrène à 12 mg/l et 50 µg/l d'anticorps anti-TFPI (T4E2).

On ajoute à 50 µl de ce mélange :
■ 25 µl d'une solution comprenant du facteur X à 80 PEU/mg/ml (flacon lyophilisé repris par de l'eau purifiée : concentration finale 8 PEU/ml).
■ 25 µl d'une solution comprenant du facteur VII à 137 PEU/mg/ml (flacon lyophilisé repris par de l'eau purifiée : concentration finale 13.7 PEU/mg/ml).
■ 50 µl d'une solution de CaCl₂ 25 mM.

Ce milieu réactionnel est incubé à 37°C pendant 500 secondes.

Après l'étape d'incubation, on ajoute 100 µl d'une solution comprenant le substrat CBS 5244 à une concentration de 0,84 µM soit une concentration finale de 3.36µM (flacon repris par de l'eau purifiée).

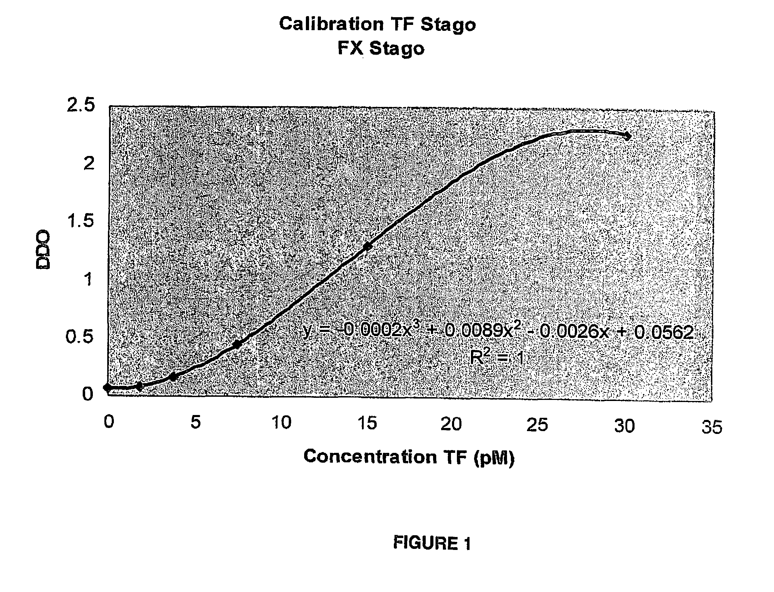

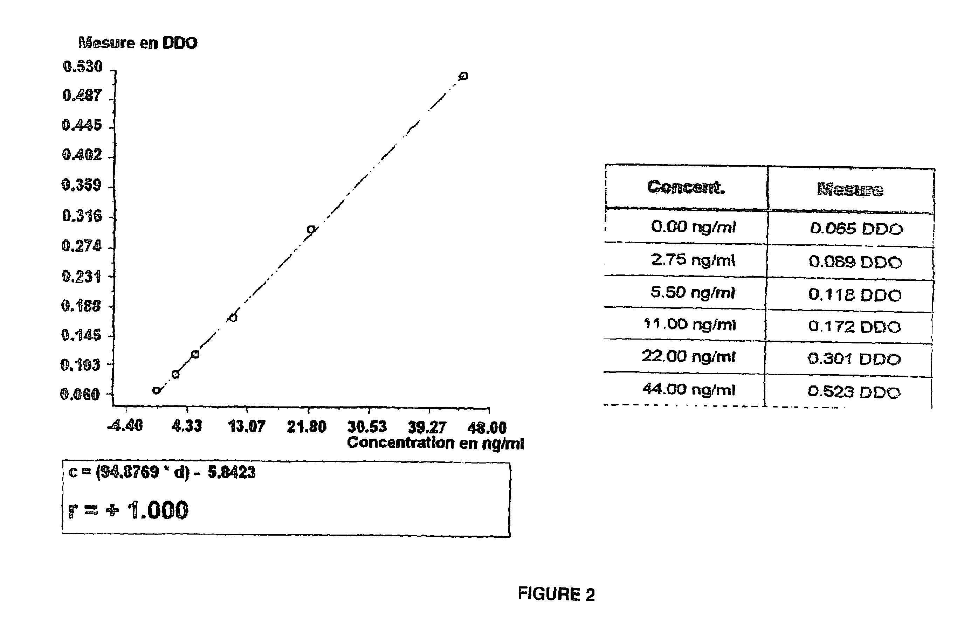

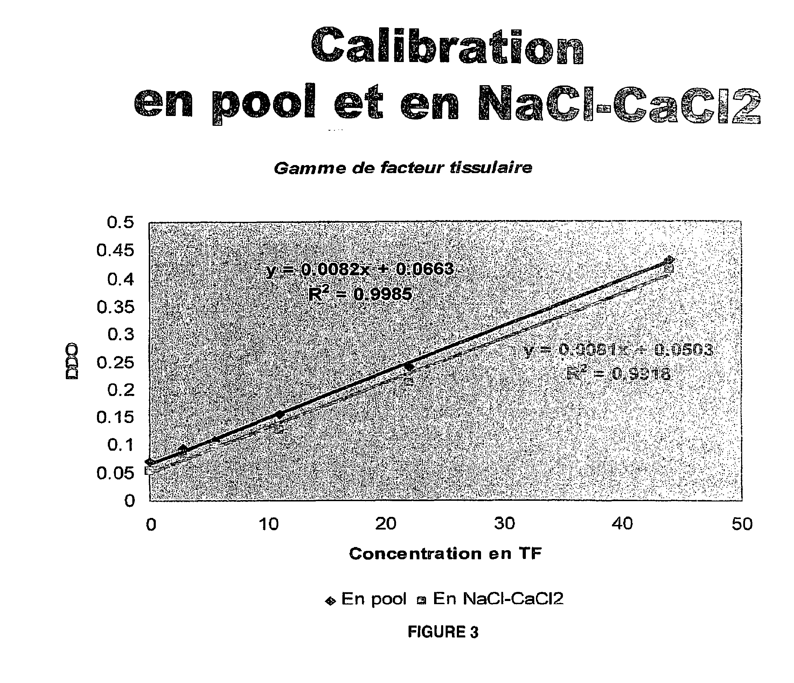

Pour établir la courbe de calibration, donnant l'activité fonctionnelle du facteur tissulaire, on a utilisé :
- soit la surcharge d'un pool de plasmas normaux par du facteur tissulaire: pour exemple en surchargeant un pool de 0.00 à 44pM de FT (Figure 3).
- soit la surcharge d'une solution NaCl-CaCl2 par du facteur tissulaire (Figure 3).

Chaque dilution de plasmas est testée au 1/3 dans la configuration.

On a déterminé la concentration FT ajoutée au pool de plasmas correspondant à une activité de 44pM, 22pM, 11 pM, 5.50pM, 2.75pM et 0pM.

L'activité du facteur tissulaire a été déterminée sur un substrat synthétique du facteur Xa, le CBS 52.44. En effet, le TF en présence de FVII va activer le FX en FXa. On a mesuré l'activité amidolytique du FXa par la libération de paranitroaniline à une longueur d'onde de 405 nm. On a déterminé la densité optique, qui correspond à chaque valeur (en pM) d'activité du facteur tissulaire, dans une fenêtre de lecture allant de 6 à 600 secondes à une longueur d'onde de 405 nm.

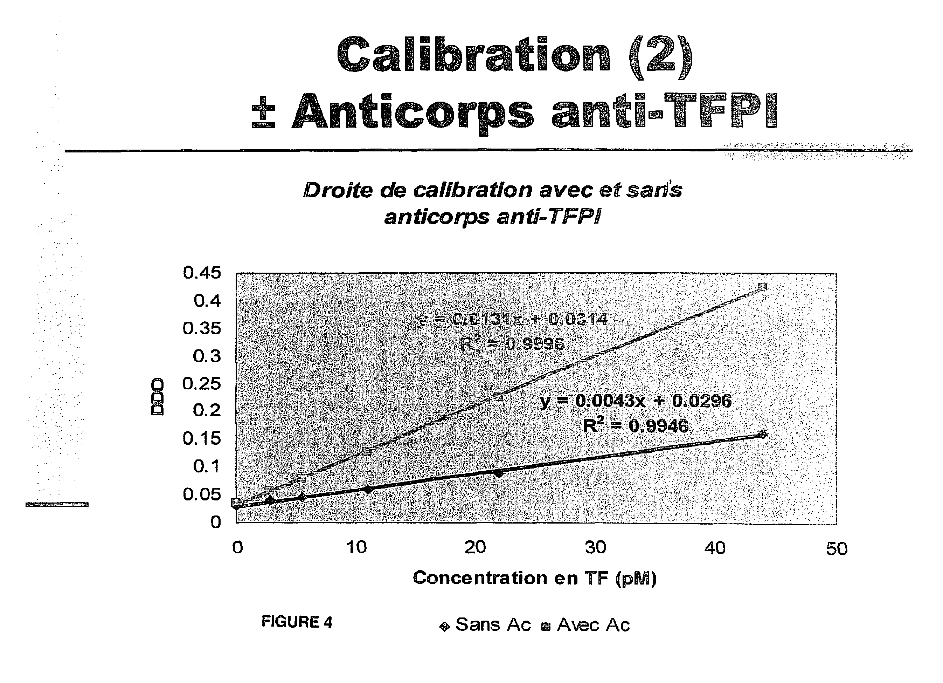

On a ensuite testé l'effet des anticorps anti-TFPI sur la calibration (Figure 4 et tableau 1).

**Tableau 1**

| Calibration (1) | | | | | | |
|---|---|---|---|---|---|---|
| ± Anticorps anti-TFPI | | | | | | |
| **TF (pM)** | **44** | **22** | **11** | **5.5** | **2.75** | **0** |
| Sans Anticorps | 0.161 | 0.088 | 0.059 | 0.046 | 0.041 | 0.031 |
| Avec Anticorps | 0.427 | 0.224 | 0.127 | 0.080 | 0.057 | 0.035 |

L'invention a également pour objet un kit pour la mesure de l'activité du facteur tissulaire circulant, en milieu biologique dans un échantillon biologique, notamment un échantillon de plasma, comprenant :
- un réactif 1 comprenant un tampon de dilution pour l'échantillon testé, un inhibiteur de la polymérisation de la fibrine, un inhibiteur du TFPI et le cas échéant un inhibiteur de l'héparine;
- du Facteur VII purifié ou recombinant; avantageusement lyophilisé ;
- du Facteur X purifié ou recombinant; avantageusement lyophilisé
- des ions calcium par exemple sous forme de CaCl₂ ;
- un substrat enzymatique du facteur X activé, avantageusement lyophilisé ;
- le cas échéant un calibrant, avantageusement lyophilisé.

L'invention a également pour objet une méthode de détection *in vitro* d'une anomalie de la coagulation sanguine, comprenant la mesure de l'activité du facteur tissulaire circulant, telle que décrite dans la présente demande. La mesure peut être réalisée dans le cadre de situations physiopathologiques diverses.

Il peut s'agir notamment de déterminer le potentiel thrombogène d'un patient susceptible de développer une thrombose, et donc sujet à une libération anormale de facteur tissulaire traduite par une activité augmentée de ce facteur vis-à-vis du facteur X.

Par ailleurs, diverses études ont été réalisées pour évaluer le rôle de plusieurs marqueurs hémostatiques durant la grossesse, afin de différencier des situations de prééclampsie des grossesses normales.

Comme cela est rapporté dans les exemples ci-après, il apparaît que l'activité du facteur tissulaire mesurée dans le plasma de femmes ayant une prééclampsie est plus élevée que celle mesurée chez des femmes ayant une grossesse normale et chez des femmes n'étant pas enceintes.

Le facteur tissulaire peut donc constituer un nouveau marqueur biologique pour évaluer les dommages subis par l'endothélium vasculaire dans des cas de prééclampsie.

L'invention décrite permet ainsi avantageusement une méthode pour évaluer le risque d'une situation de prééclampsie dans le cas d'une grossesse, dans laquelle on mesure l'activité du TF circulant selon les caractéristiques de l'invention. Ce document décrit également un kit pour la mise en oeuvre de ladite méthode.

De même, comme cela l'est également indiqué dans les exemples ci-après, le dosage de l'activité du TF, et notamment le rapport TF activé / TFPI libre, fournit un nouvel outil pour le suivi d'évolution des pathologies, par exemple pour le suivi d'un infarctus du myocarde aïgu (AMI), pathologie dans laquelle à la fois le taux de TF activé et le ratio TFactivé/TFPI libre sont augmentés par rapport aux contrôles, et pour l'établissement d'un pronostic favorable ou pas quant à l'évolution de cette pathologie.

Ce document décrit donc une méthode pour le suivi ou l'établissement d'un pronostic d'un infarctus du myocarde aigu dans laquelle on mesure l'activité du TF circulant ou le ratio TF circulant / TFPI libre selon les caractéristiques de l'invention. Ce document décrit également un kit pour la mise en oeuvre de ladite méthode.

Enfin, comme le montrent des données récentes présentées par exemple dans des congrès internationaux (ASH Meeting, Atlanta USA, 7-11 Décembre 2007, Thrombosis and Haemostasis Issues in Cancer, Bergame, Italie 26-28 Octobre 2007), l'implication du facteur tissulaire dans l'apparition et le développement de cancers est désormais bien reconnue. Sur un plan diagnostique, le dosage de l'activité du TF, notamment d'une augmentation de celle-ci, est un nouvel indicateur pour le suivi de l'évolution de pathologies cancéreuses.

Ce document décrit ainsi avantageusement une méthode pour le suivi de l'évolution de pathologies cancéreuses, dans laquelle on mesure l'activité du TF circulant selon les caractéristiques de l'invention. Ce document décrit également un kit pour la mise en oeuvre de ladite méthode.

Le dosage de l'activité du facteur tissulaire pourrait donc être un plus dans l'évaluation et le pronostique de différentes pathologies associées a la grossesse en particulier pour l'évaluation des risques de fausse couche et de complications de la grossesse telles que la pré-éclampsie, aux thromboses, aux sepsis, aux cancers, aux inflammations, aux CIVD, au diabète, aux thrombopénies mais également ce dosage pourrait avoir un intérêt dans l'évaluation des traitements antithrombotiques comme par exemple les traitements au TFPI.

Les figures et les exemples ci-après illustrent la présente invention.

Dans le but d'estimer la valeur normale d'un plasma, 31 plasmas normaux ont été dosés pour l'activité du facteur tissulaire, suivant la méthode de l'invention. Les mesures de densité optique et les pourcentages d'activité FT correspondants sont rapportés dans le tableau ci-dessous. Ces mesures peuvent être considérées comme indicatrices des valeurs normales d'activité fonctionnelle du facteur tissulaire dans des plasmas normaux.

**Plasmas normaux**

| | **Normaux n = 31** | |
|---|---|---|
| | **DDO** | **pM** |
| **Moyenne** | 0.038 | 0.79 |
| **Médiane** | 0.037 | 0.72 |
| **Min** | 0.025 | < 0.10 |
| **Max** | 0.055 | 1.15 |

La sensibilité à l'héparine a été évaluée en ajoutant a un plasma surchargé en FT (22pM), dosé dans les conditions de dosage décrites ci-dessus, des concentrations croissantes de calciparine (0-2Ul/ml). L'insensibilité du test à l'héparine montre un recouvrement des taux > à 94% (tableau 2). On montre bien que la calciparine s'avère peu déterminante sur l'activité fonctionnelle du FT.

**Tableau 2**

| Sensibilité à l'héparine L'étude de sensibilité a été réalisée sur une gamme de calciparine en Pool + TF 22pM | | | |
|---|---|---|---|
| | ***DDO*** | **pM** | ***Recouvrement*** |
| Pool - TF 22pM | 0.207 | 22 | 100% |
| Calciparine 0.4 | 0.189 | 20.6 | 94% |
| Calciparine 1.0 | 0.199 | 22 | 100% |
| Calciparine 1.4 | 0.204 | 22 | 100% |
| Calciparine 2.0 | 0.203 | 22 | 100% |

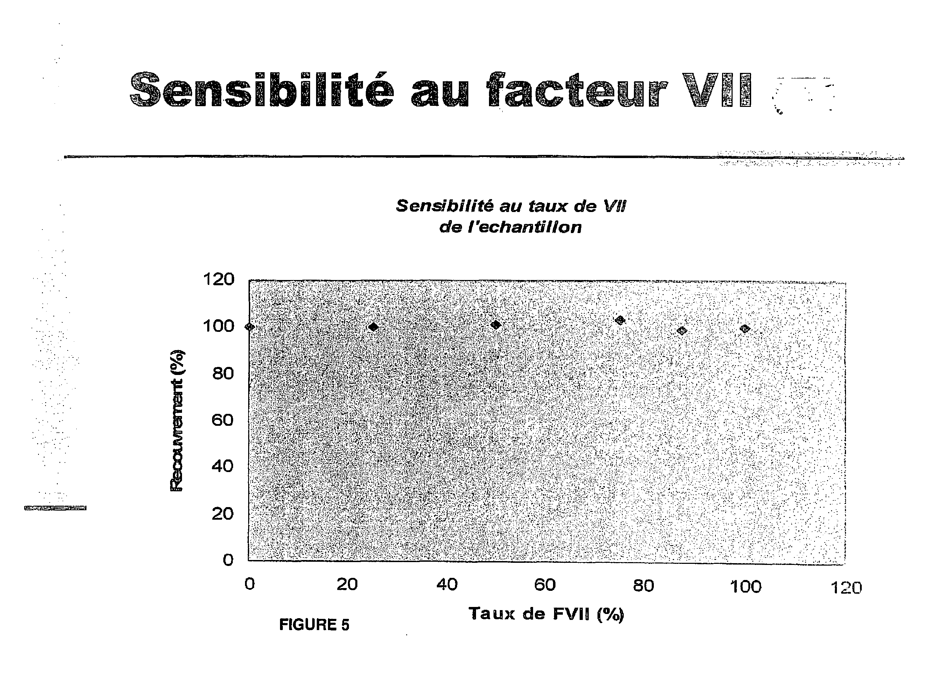

Des plasmas surchargés en FT comprenant une concentration variable déterminée en facteur VII ont été dosés selon la méthode décrite ci-dessus.
Il s'agit de:
- Plasma déficient en facteur VII (Colonne 0%)
- Plasmas d'un pool rendus plus ou moins déficients en facteur VII (colonnes 25 - 87.5%)
- Plasma d'un pool normal (colonne 100%).
Les résultats (Figure 5 et tableau 3) montrent avec des recouvrements ≥ 99% que le taux de facteur VII de l'échantillon n'influence pas la valeur de l'activité fonctionnelle du FT.

**Tableau 3**

| Sensibilité au facteur VII (1) | | | | | | |
|---|---|---|---|---|---|---|
| **Taux de VII %** | **0** | **25** | **50** | **75** | **87.5** | **100** |
| Tx théorique TF (pM) | 39.97 | 40.66 | 41.36 | 42.05 | 42.41 | 42.76 |
| Tx mesurée TF (pM) | 39.97 | 40.66 | 41.88 | 43.58 | 42.07 | 42.76 |
| **Recouvrement %** | **100** | **100** | **101** | **104** | **99** | **100** |

Sensibilité réalisée à partir d'un déficient VII et d'un pool surchargé en facteur tissulaire.
0 : Déf VII- Pool-TF
25 - 87.5% : Pool-TF/Déf VII-TF
100%: Pool-TF

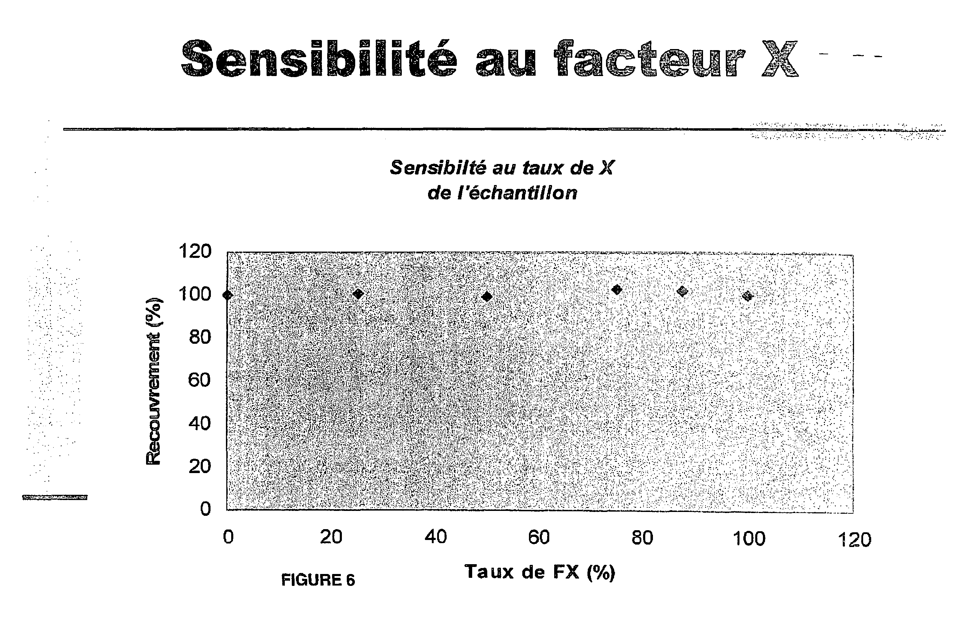

Des plasmas surchargés en FT comprenant une concentration variable déterminée en facteur X ont été dosés selon la méthode décrite ci-dessus.
Il s'agit de:
- Plasma déficient en facteur X (Colonne 0%)
- Plasmas d'un pool rendus plus ou moins déficients en facteur X (colonnes 25 - 87.5%)
- Plasma d'un pool normal (colonne 100%).
Les résultats (Figure 6 et tableau 4) montrent avec des recouvrements > 99% que le taux de facteur X de l'échantillon n'influence pas la valeur de l'activité fonctionnelle du FT.

**Tableau 4**

| Sensibilité au facteur X (1) | | | | | | |
|---|---|---|---|---|---|---|
| **Taux de X %** | **0** | **25** | **50** | **75** | **87.5** | **100** |
| Tx théorique TF pM | 45.57 | 44.86 | 44.16 | 43.45 | 43.47 | 42.76 |
| Tx mesurée TF pM | 45.57 | 45.10 | 43.82 | 44.63 | 44.41 | 42.76 |
| **Recouvrement** | **100** | **101** | **99** | **103** | **102** | **100** |

Sensibilité réalisée à partir d'un déficient X et d'un pool surchargé en facteur tissulaire.
0 : Déf X-TF
25 - 87.5% : Pool-TF / Déf X-TF
100%: Pool-TF

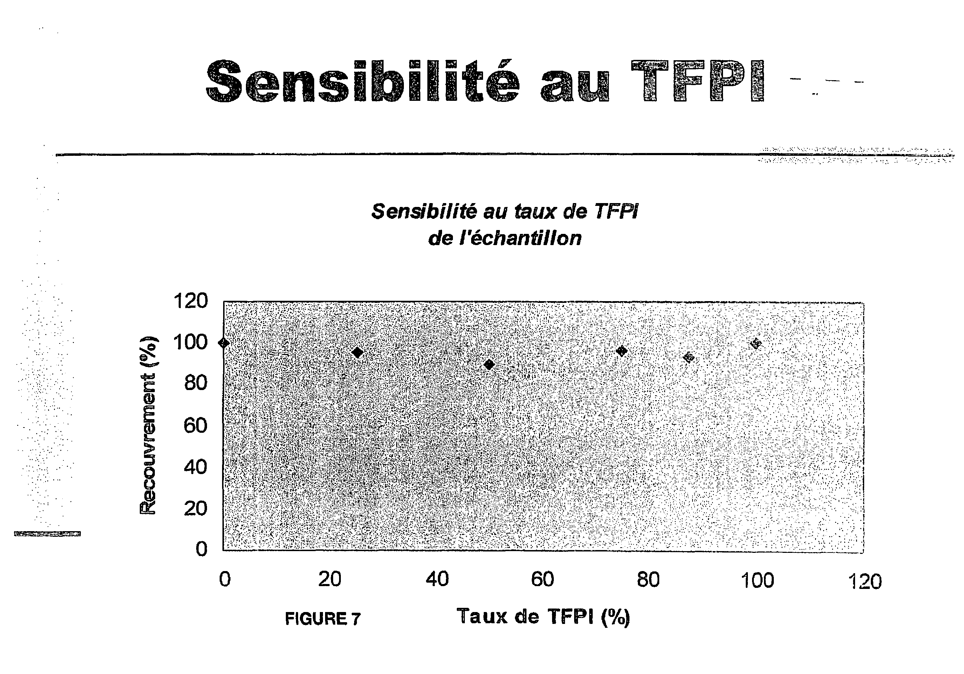

Des plasmas surchargés en FT comprenant une concentration variable déterminée en TFPI ont été dosés selon la méthode décrite ci-dessus.
Il s'agit de:
- Plasma déficient en TFPI (Colonne 0%)
- Plasmas d'un pool rendus plus ou moins déficients en facteur TFPI (colonnes 25 - 87.5%)
- Plasma d'un pool normal (colonne 100%).
Les résultats (Figure 7 et tableau 5) montrent avec des recouvrements > 93% que le taux de TFPI de l'échantillon n'influence pas la valeur de l'activité fonctionnelle du FT.

**Tableau 5**

| Sensibilité au TFPI (1) | | | | | | |
|---|---|---|---|---|---|---|
| **Taux de TFPI %** | **0** | **25** | **50** | **75** | **87.5** | **100** |
| Tx théorique TF pM | 48.25 | 46.86 | 45.50 | 44.13 | 43.44 | 42.76 |
| Tx mesurée TF pM | 48.25 | 44.86 | 40.77 | 42.54 | 40.55 | 42.76 |
| **Recouvrement %** | **100** | **96** | **90** | **96** | **93** | **100** |

Sensibilité réalisée à partir d'un déficient TFPI et d'un pool surchargé en facteur tissulaire.
0 : Déf TFPI-TF
25 - 87.5% : Pool-TF / Déf TFPI-TF
100% : Pool-TF

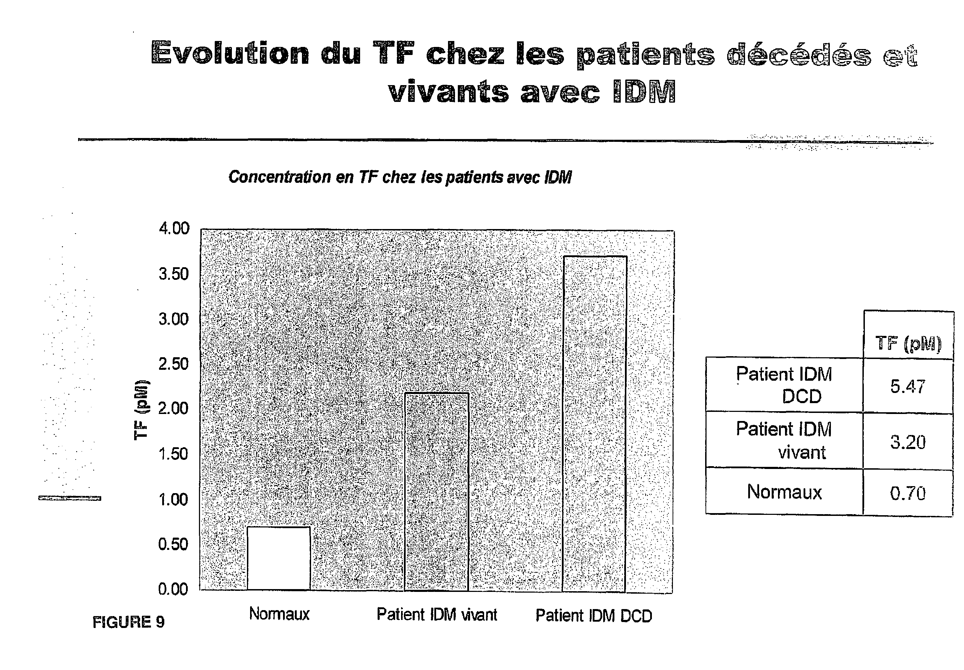

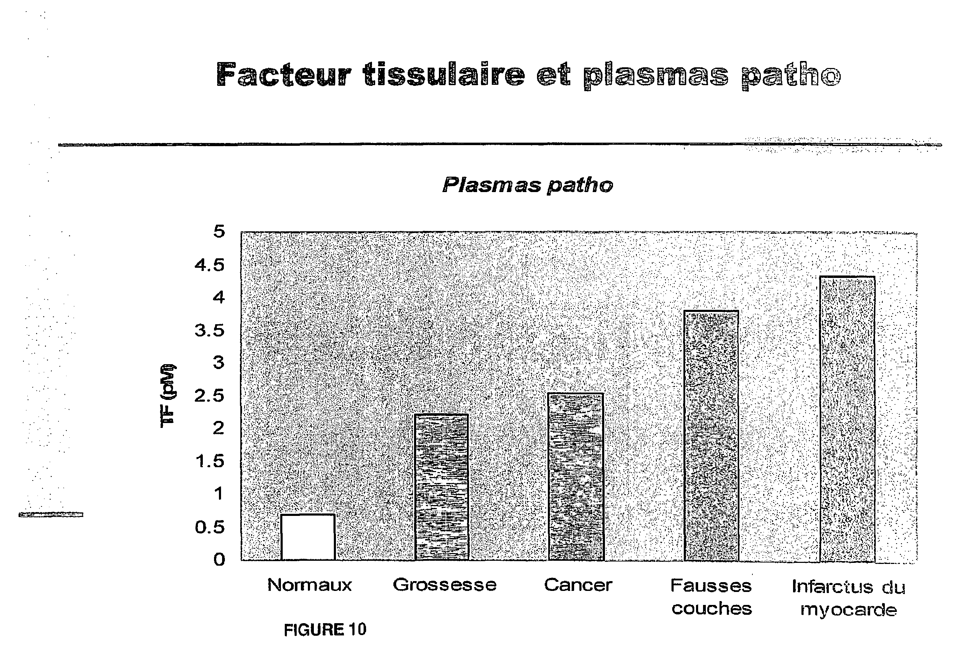

A titre d'exemples dans différentes pathologies,
- la mesure de l'activité du FT a été réalisée sur un groupe de sujets ne présentant aucune pathologie et au jour de l'admission d'un groupe de patients apparenté souffrant d'infarctus du myocarde (IDM), ce groupe se divisant en sujets à bon pronostique et patients décédés dans les jours suivant l'infarctus ( IDM DCD), les taux en FT sont significativement plus forts chez les IDM que les sujets normaux, et nettement supérieurs chez les patients décédés (Figure 9).
- L'activité du FT circulant a également été dosée dans d'autres pathologies thrombotiques comme les cancers, les fausses couches : 3,65 ± 1,35 à 3,92 ± 1,29 selon le stade de perte du foetus, thrombopénies induites par l'héparine (TIH) ; 6,33±2,10 pM, les CIVD : 2,81 ± 1,52 pM, le diabéte en crise : 2,98 ± 0,99 pM et lupus : 1,95±0,82 pM ou lors de la grossesse notamment lors de complications de la grossesse comme la pré-éclampsie : 3,58 ± 1,34 pM comparé à 1,98 ± 1,25 pM lors d'une grossesse normale ; toutes ces determinations montrent une augmentation significative de l'activité du FT (Figure 10).
- Mesure de répétabilité
La répétabilité des mesures a été testée et conduit aux valeurs suivantes :

| | ***TIH*** | |
|---|---|---|
| | ***DDO*** | ***pM*** |
| **Moyenne** | **0.186** | **16.19** |
| **Min** | **0.18** | **15.54** |
| **Max** | **0.2** | **17.72** |
| **Ecart-type** | **0.005** | **0.528** |
| **CV** | **2.62** | **3.26** |

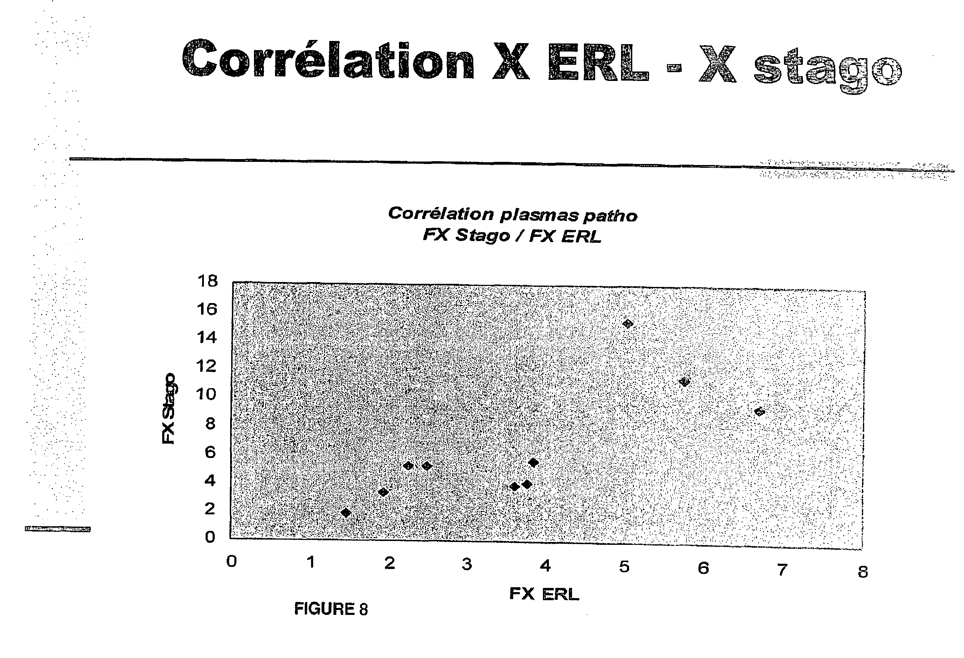

- La corrélation des mesures de facteur X (ERL et Stago) sur des plasmas pathologiques a donné les résultats suivants illustrés à la figure 8.

| Corrélation X ERL - X stago (1) | | | | |
|---|---|---|---|---|
| | **FX ERL** | | **FX Stago** | |
| | **DDO** | **pM** | **DDO** | **pM** |
| Patho 1 | 0.088 | 2.85 | 0.074 | 4.91 |
| Patho 2 | 0.092 | 3.30 | 0.085 | 7.73 |
| Patho 3 | 0.127 | 7.39 | 0.143 | 22.6 |
| Patho 4 | 0.109 | 5.29 | 0.077 | 5.67 |
| Patho 5 | 0.095 | 3.66 | 0.085 | 7.73 |
| Patho 6 | 0.136 | 8.45 | 0.12 | 16.73 |
| Patho 7 | 0.112 | 5.64 | 0.087 | 8.25 |
| Patho 8 | 0.082 | 2.14 | 0.066 | 2.85 |
| Patho 9 | 0.111 | 5.53 | 0.078 | 5.94 |
| Patho 10 | 0.148 | 9.85 | 0.109 | 13.91 |

- Le taux (en pM) du facteur tissulaire de la société American Diagnostic a été mesuré suivant l'invention.

Les résultats sont lus sur une gamme réalisée à partir de Neo-R Stago (test effectué en simple) en se basant sur une concentration en FT de 6,2 nM dans le flacon de Neo-R après reconstitution

| | **DDO** | **pM** |
|---|---|---|
| Pool + TF A.D 30pM | 0.259 | 23 |
| Pool + TF A.D. 15pM | 0.175 | 13 |
| Pool + TF A.D. 7.5pM | 0.113 | 5.9 |
| Pool + TF A.D. 3.75pM | 0.089 | 3.0 |
| Pool + TF A.D. 1.88pM | 0.073 | 1.1 |

### BIBLIOGRAPHIE

1. Valéry Daubie, Roland Pochet, Sophie Houard and Pierre Philippart. Tissue factor : a mini-review. J. Tissue Eng Regen Med. 2007 ; 1 : 161-169.
2. Rüdiger Gerlach, Timm Scheuer, Martina Böhm, Jürgen Beck, Alina Woszczyk, Andreas Raabe, Inge Scharrer and Volker Seifert. Increased levels of plasma tissue factor pathway inhibitor in patients with glioblastoma and intracerebral metastases. Neurological Research. 2003 ; Vol 25, 335-338.
3. Anat Aharon, Benjamin Brenner, Tamar Katz, Yohei Miyagi, Naomi Lanir. Tissue factor and tissue factor pathway inhibitor levels in trophoblast cells : implications for placental hemostasis. Thromb. Haemost. 2004 ; 92 : 776-86.
4. Nigel Mackman. Role of tissue factor in hemostasis and thrombosis Blood cells Molecules and Diseases 2006; 36: 104-107.
5. Valéry Daubie, Roland Pochet, Sophie Houard, Pierre Philippart. Tissue factor: a mini-review J Tissue Eng Regen Med 2007; 1: 161-169.
6. Rachel Tilley, Nigel Mackman. Tissue factor in hemostasis and thrombosis. Semin Thromb and Hemost 2006; 31 : 5-10.
7. Bjarne Osterud, Eirik Bjorklid. Sources of tissue factor. Semin Thromb and Hemost 2006; 32: 11-23.
8. Arthur J. Chu. Tissue factor mediates inflammation. Archives of Biochem and Biophy 2005; 440: 123-132.
9. Janusz Rak, Chloe Milson, Linda May, Petr Klement, Joanne Yu. Tissue factor in cancer and angiogenesis: The molecular link between genetic timor progression, tumor neovascularization, and cancer coagulopathy. Semin Thromb and Hemost 2006; 32: 54-70
10. Pierre-Francois Laterre, Xavier Wittebole, Christine Collienne. Pharmacological inhibition of tissus factor. Semin Thromb and Hemost 2006; 32: 71-76

## Revendications

1. Méthode de dosage *in vitro* de l'activité du facteur tissulaire circulant (FTc) dans un échantillon biologique dérivé d'un prélèvement préalablement fait chez un patient, tel qu'un échantillon de sang ou un dérivé, notamment un échantillon de plasma, dans laquelle on mesure la génération de facteur X activé (Xa) dans un milieu réactionnel comprenant l'échantillon biologique testé, un excès de facteur VII et de facteur X, des ions calcium, par exemple apportés sous forme de CaCl₂, un inhibiteur de la polymérisation de la fibrine et un inhibiteur de l'activité du TFPI (Tissue Factor Pathway Inhibitor).

2. Méthode selon la revendication 1, **caractérisée en ce que** la génération du facteur Xa dans le milieu réactionnel est déterminée par la mesure de l'activité d'hydrolyse dudit facteur sur un substrat spécifique.

3. Méthode selon la revendication 2, **caractérisée en ce que** le substrat spécifique est un substrat enzymatique ou que l'activité amidolytique du facteur X activé (FXa) est dosée au moyen d'un substrat chromogénique ou fluorométrique, par exemple en dosant la libération de paranitroaniline par le substrat chromogène synthétique CBS 5244.

4. Méthode selon l'une quelconque des revendications 1 à 3, **caractérisée en ce que** le milieu réactionnel comprend du plasma dilué dans un tampon approprié, par exemple un tampon Owren Koller, en particulier dans un intervalle de dilution de 1/2 à 1/20, par exemple de 1/2 à 1/4, en particulier le plasma est dilué à 1/3.

5. Méthode selon l'une quelconque des revendications 1 à 4, **caractérisée en ce que** l'inhibiteur de la polymérisation de la fibrine est un inhibiteur polypeptidique, par exemple le *H*-GlyProArgPro-*OH.AcOH* (GPRP.AcOH) ou est un anticorps anti-fibrinogène.

6. Méthode selon l'une quelconque des revendications 1 à 5, **caractérisée en ce que** l'inhibiteur de l'activité du TFPI sur le facteur tissulaire circulant est constitué d'anticorps anti-TFPI, ou de fragments fonctionnels d'anticorps liant le TFPI capables d'inhiber l'activité du TFPI sur le facteur tissulaire circulant, par exemple un anticorps monoclonal T4E2 ou un fragment fonctionnel de cet anticorps.

7. Méthode selon l'une quelconque des revendications 1 à 6, comprenant les étapes de :
a) mise en contact d'un échantillon de plasma avec un réactif 1 comprenant un inhibiteur de la polymérisation de la fibrine, un inhibiteur de l'activité du TFPI sur le FTc et, le cas échéant, un tampon de dilution et/ou un inhibiteur de l'héparine, par exemple le polybrène;
b) incubation du milieu réactionnel constitué à l'étape a) avec un réactif 2 comprenant du facteur X en excès, du facteur VII en excès et des ions calcium en solution, de façon à produire du facteur X activé (FXa) ;
c) après incubation, la mise en contact du milieu réactionnel constitué à l'étape b) avec un substrat enzymatique du facteur X activé (FXa);
d) la détection de la transformation du substrat du facteur Xa.

8. Méthode selon la revendication 7, **caractérisée en ce que** l'échantillon de plasma est dilué au 1/3, dans un tampon Owren Koller additionné de polybrène à 1,2 mg/l et de 50µg/l d'anticorps anti-TFPI.

9. Méthode selon l'une quelconque des revendications 7 ou 8, **caractérisée en ce que** la mesure quantitative de la transformation du substrat enzymatique du facteur Xa se fait par lecture de densité optique dans une fenêtre déterminée.

10. Méthode selon l'une des revendications 7 à 9, **caractérisée en ce que** l'on dose la quantité de substrat transformé du facteur Xa par référence à une courbe de calibration.

11. Méthode selon l'une quelconque des revendications 7 à 10, **caractérisée en ce que** la réaction est effectuée dans les conditions suivantes :
- pour l'étape a), on met en contact l'échantillon de plasma dilué au 1/3 dans un tampon Owren Koller, avec 50µg/l d'anticorps anti-TFPI et du polybrène à 1,2mg/L et un inhibiteur de la polymérisation de la fibrine désigné GPRP.AcOH à une concentration de 10g/L ;
- pour l'étape b) on ajoute à 50 µl du mélange de l'étape a), 25 µl de facteur X à 80 Plasma Equivalent Unit (PEU)/mg/ml, 25µl de facteur VII à 137 Plasma Equivalent Unit (PEU)/mg/ml et 50µl d'une solution de CaCl₂ 25mM et on incube le mélange réactionnel obtenu à 37°C pendant 500 secondes ;
- pour l'étape c) on ajoute 100 µl d'une solution de substrat enzymatique du Facteur X activé, et on détermine la quantité de substrat enzymatique transformé, par lecture optique dans une fenêtre de lecture de 6 à 600 s, à une longueur d'onde de 405 nm.

12. Méthode selon l'une quelconque des revendications 1 à 11, **caractérisée en ce qu'**elle comprend en outre le dosage d'un contrôle interne.

13. Méthode selon l'une quelconque des revendications 1 à 12, **caractérisée en ce que** les valeurs de densité optique obtenues sont comparées à celles d'une courbe de calibration préparée au moyen d'un pool de plasmas normaux surchargés en facteur tissulaire ou au moyen d'une solution de NaCl-CaCl₂ surchargée en facteur tissulaire.

14. Kit pour la mesure de l'activité du facteur tissulaire circulant, en milieu biologique dans un échantillon biologique, notamment dans un échantillon de plasma, comprenant :
- un réactif 1 comprenant un tampon de dilution pour l'échantillon testé, un inhibiteur de la polymérisation de la fibrine, un inhibiteur du TFPI et le cas échéant un inhibiteur de l'héparine;
- du Facteur VII purifié ou recombinant ; avantageusement lyophilisé ;
- du Facteur X purifié ou recombinant ; avantageusement lyophilisé
- des ions calcium par exemple sous forme de CaCl₂ ;
- un substrat enzymatique du facteur X activé, avantageusement lyophilisé ;
- le cas échéant un calibrant, avantageusement lyophilisé.

15. Kit selon la revendication 14, **caractérisé en ce que** le tampon de dilution de l'échantillon est un tampon Owren Kroller, l'inhibiteur de l'héparine est le polybrène et l'inhibiteur de la polymérisation de la fibrine est le *H*-GlyProArgPro-*OH.AcOH* (GPRP.AcOH) ou est un anticorps anti-fibrinogène.

16. Kit selon l'une quelconque des revendications 14 ou 15 **caractérisé en ce qu'**il comprend en outre un contrôle interne et/ou une courbe de calibration de l'activité du facteur Xa.

17. Méthode de détection *in vitro* d'une anomalie de la coagulation, comprenant la mesure de l'activité du facteur tissulaire circulant selon l'une quelconque des revendications 1 à 13 et optionnellement la comparaison du niveau de l'activité mesurée du facteur tissulaire circulant, à une valeur normale de cette activité.

18. Méthode selon la revendication 17, dans laquelle la valeur normale est obtenue après dosage de l'activité du facteur tissulaire circulant sur un pool d'échantillons de plasma normaux ou établie à partir de valeurs de l'activité du facteur tissulaire circulant mesurées sur des échantillons de plasma normaux dosés individuellement.

19. Méthode selon l'une quelconque des revendications 17 à 18 ou kit selon l'une quelconque des revendications 14 à 16, pour la détection d'une augmentation du taux de facteur tissulaire circulant associée à un risque de thrombose ou à une thrombose et en particulier à des symptômes d'infarctus aïgu du myocarde (AMI), de syndrome coronaire aïgu, de coagulation intravasculaire disséminée (CIVD), de diabète, de prééclampsie, de fausse couche, de thrombose, de sepsis, d'inflammation, de cancer.

## Patentansprüche

1. Verfahren zum in vitro-Dosieren der Aktivität des zirkulierenden Gewebefaktors (FTc) in einer biologischen Probe, die aus einer zuvor bei einem Patienten gemachten Entnahme stammt, wie einer Blutprobe oder einem Derivat, insbesondere einer Plasmaprobe, in der man die Bildung des aktivierten Faktors X (Xa) in einem Reaktionsmedium umfassend die getestete biologische Probe, einen Überschuss des Faktors VII und des Faktors X, Calciumionen, zum Beispiel in Form von CaCl₂, einen Polymerisationsinhibitor von Fibrin und einen Inhibitor der Aktivität von TFPI (Tissue Factor Pathway Inhibitor), misst.

2. Verfahren nach Anspruch 1, **dadurch gekennzeichnet, dass** die Bildung des Faktors Xa in dem Reaktionsmedium durch die Messung der Aktivität der Hydrolyse des Faktors auf einem spezifischen Substrat bestimmt wird.

3. Verfahren nach Anspruch 2, **dadurch gekennzeichnet, dass** das spezifische Substrat ein enzymatisches Substrat ist oder dass die amidolytische Aktivität des aktivierten Faktors X (FXa) anhand von mindestens einem chromogenen oder fluorometrischen Substrat dosiert wird, zum Beispiel durch Dosieren der Freisetzung des Paranitroanilins anhand von dem synthetischen chromogenen Substrat CBS 5244.

4. Verfahren nach einem beliebigen der vorhergehenden Ansprüche 1 bis 3, **dadurch gekennzeichnet, dass** das Reaktionsmedium in einem geeigneten Puffer verdünntes Plasma umfasst, zum Beispiel einem Owren Koller Puffer, insbesondere in einem Verdünnungsintervall von 1/2 bis 1/20, zum Beispiel von 1/2 bis 1/4, insbesondere ist das Plasma auf 1/3 verdünnt.

5. Verfahren nach einem beliebigen der vorhergehenden Ansprüche 1 bis 4, **dadurch gekennzeichnet, dass** der Polymerisationsinhibitor des Fibrins ein polypeptidischer Inhibitor ist, zum Beispiel der H-GlyProArgPro-OH.AcOH (GPRP.AcOH) oder ein anti-fibrinogener Antikörper ist.

6. Verfahren nach einem beliebigen der vorhergehenden Ansprüche 1 bis 5, **dadurch gekennzeichnet, dass** der Inhibitor der Aktivität von TFPI auf den zirkulierenden Gewebefaktor aus Antikörper anti-TFPI oder den funktionellen Antikörperfragmenten, die den TFPI verbinden, die fähig sind die Aktivität des TFPI auf den zirkulierenden Gewebefaktor zu inhibieren, besteht, zum Beispiel aus einem monoklonalen Antikörper T4E2 oder einem funktionellen Fragment dieses Antikörpers.

7. Verfahren nach einem beliebigen der vorhergehenden Ansprüche 1 bis 6, umfassend die Schritte von:
a) In-Kontakt-Bringen einer Plasma probe mit einem Reagens 1, umfassend einen Polymerisationsinhibitor des Fibrins, einen Inhibitor der Aktivität des TFPI auf den FTc und, gegebenenfalls einen Verdünnungspuffer und/oder einen Inhibitor des Heparins, zum Beispiel das Polybren;
b) Inkubieren des in Schritt a) gebildeten Reaktionsmediums mit einem Reagens 2, umfassend den Faktor X im Überschuss, den Faktor VII im Überschuss und Calciumionen in Lösung, sodass aktivierter Faktor X (FXa) hergestellt wird;
c) nach dem Inkubieren, In-Kontakt-Bringen des in Schritt b) gebildeten Reaktionsmediums mit einem enzymatischen Substrat des aktivierten Faktors X (FXa);
d) Detektieren der Transformation des Substrats des Faktors Xa.

8. Verfahren nach Anspruch 7, **dadurch gekennzeichnet, dass** die Plasmaprobe auf 1/3 verdünnt wird, in einem Owren Koller-Puffer, versetzt mit Polybren bei 1,2 mg/L und 50 µg/L Antikörpern anti-TFPI.

9. Verfahren nach einem beliebigen der vorhergehenden Ansprüche 7 oder 8, **dadurch gekennzeichnet, dass** die quantitative Messung der Transformation des enzymatischen Substrats des Faktors Xa durch Lesen der optischen Dichte in einem bestimmten Fenster durchgeführt wird.

10. Verfahren nach einem beliebigen der vorhergehenden Ansprüche 7 bis 9, **dadurch gekennzeichnet, dass** man die Menge an transformiertem Substrat des Faktors Xa, bezogen auf die Kurve der Kalibrierung, dosiert.

11. Verfahren nach einem beliebigen der vorhergehenden Ansprüche 7 bis 10, **dadurch gekennzeichnet, dass** die Reaktion unter den folgenden Bedingungen durchgeführt wird:
- für Schritt a) bringt man die Plasma probe verdünnt auf 1/3 in einen Owren Koller Puffer mit 50 µg/L Antikörpern anti-TFPI und Polybren bei 1,2 mg/L und einen Polymerisationsinhibitor des Fibrins, bezeichnet GPRP.AcOH, bei einer Konzentration von 10 g/L in Kontakt;
- für Schritt b) fügt man 50 µl des Gemischs von Schritt a) hinzu, 25 µl des Faktors X auf 80 Plasma-Äquivalenteinheiten (Plasma Equivalent Unit), PEU/mg/ml, 25 µl des Faktors VII auf 137 Plasma-Äquivalenteinheiten (PEU/mg/ml) und 50 µl einer CaCl₂-Lösung 25 mM, und man inkubiert die erhaltene Reaktionsmischung bei 37 °C während 500 Sekunden;
- für Schritt c) fügt man 100 µl einer Lösung des enzymatischen Substrats des aktivierten Faktors X hinzu, und man bestimmt die Menge des transformierten enzymatischen Substrats, durch optisches Lesen in einem Auslesungsfenster von 6 bis 600 s, bei einer Wellenlänge von 405 nm.

12. Verfahren nach einem beliebigen der vorhergehenden Ansprüche 1 bis 11, **dadurch gekennzeichnet, dass** das Verfahren unter anderem die Dosierung einer internen Kontrolle umfasst.

13. Verfahren nach einem beliebigen der vorhergehenden Ansprüche 1 bis 12, **dadurch gekennzeichnet, dass** die Werte der erhaltenen optischen Dichte mit einer Kalibrierungskurve, die mit Hilfe eines Pools von normalen Plasmen überladen mit Gewebefaktor oder mit Hilfe einer Lösung von NaCl-CaCl₂ überladen mit Gewebefaktor verglichen werden.

14. Kit zum Messen der Aktivität des zirkulierenden Gewebefaktors, in biologischem Medium in einer biologischen Probe, insbesondere in einer Plasmaprobe, umfassend:
- ein Reagens 1 umfassend einen Verdünnungspuffer für die getestete Probe, einen Polymerisationsinhibitor des Fibrins, einen Inhibitor des TFPI und gegebenenfalls einen Inhibitor von Heparin,
- den gereinigten oder rekombinierten Faktor VII, vorteilhafterweise lyophilisiert,
- den gereinigten oder rekombinierten Faktor X, vorteilhafterweise lyphilisiert;
- Calciumionen, zum Beispiel in Form von CaCl₂,
- ein enzymatisches Substrat des aktivierten Faktors X, vorteilhafterweise lyophilisiert;
- gegebenenfalls ein Kalibrierungsmittel, vorteilhafterweise lyophilisiert.

15. Kit nach Anspruch 14, **dadurch gekennzeichnet, dass** der Verdünnungspuffer der Probe ein Owren Koller-Puffer ist, der Inhibitor des Heparins das Polybren ist und der Polymerisationsinhibitor des Fibrins das H-GlyProArgPro-OH.AcOH (GPRP.AcOH) ist oder ein antifibrinogener Antikörper ist.

16. Kit nach einem beliebigen der vorhergehenden Ansprüche 14 oder 15, **dadurch gekennzeichnet, dass** er unter anderem eine interne Kontrolle und/oder eine Kalibrierungskurve der Aktivität des Faktors Xa umfasst.

17. Verfahren zum in vitro-Detektieren einer Koagulations-Anomalie, umfassend das Messen der Aktivität des zirkulierenden Gewebefaktors gemäß einem beliebigen der vorhergehenden Ansprüche 1 bis 13 und optional das Vergleichen des Niveaus der gemessenen Aktivität des zirkulierenden Gewebefaktors mit einem normalen Wert von dieser Aktivität.

18. Verfahren nach Anspruch 17, in welcher der normale Wert nach Dosierung der Aktivität des zirkulierenden Gewebefaktors auf einen Pool von normalen Plasmaproben erhalten wird oder ausgehend von Werten der Aktivität des zirkulierenden Gewebefaktors gemessen auf normalen Plasmaproben, die individuell dosiert wurden, bestimmt wird.

19. Verfahren nach einem beliebigen der vorhergehenden Ansprüche 17 bis 18 oder Kit nach einem beliebigen der vorhergehenden Ansprüche 14 bis 16, für die Detektion einer Erhöhung des zirkulierenden Gewebefaktorspiegels assoziiert mit einem Thromboserisiko oder mit einer Thrombose und im Besonderen den Symptomen von akutem Herzmyokardininfarkt (AMI), akutem Koronarsyndrom, disseminierter intravaskulärer Gerinnung (CIVD), Diabetes, Präeklampsie, Fehlgeburt, Thrombose, Sepsis, Entzündung, Krebs.

## Claims

1. A method for *in vitro* assay of the activity of circulating tissue factor (cTF) in a biological sample originating from the prior sampling of a patient, such as a blood sample or a derivative thereof, especially a plasma sample, wherein the generation of activated factor X (Xa) is measured in a reaction medium comprising the tested biological sample, an excess of factor VII and of factor X, calcium ions, for example supplied in the form of CaCl₂, a fibrin polymerization inhibitor and a TFPI (Tissue Factor Pathway Inhibitor) activity inhibitor.

2. A method as claimed in claim 1, **characterized in that** the generation of factor Xa in the reaction medium is determined by measuring the hydrolysis activity of said factor on a specific substrate.

3. A method as claimed in claim 2, **characterized in that** the specific substrate is an enzymatic substrate or **in that** the amidolytic activity of activated factor X (FXa) is assayed using a chromogenic or fluorometric substrate, for example by assaying the liberation of para-nitroaniline by the synthetic chromogenic substrate CBS 5244.

4. A method as claimed in any one of claims 1 to 3, **characterized in that** the reaction medium comprises plasma diluted in an appropriate buffer, for example an Owren Koller buffer, especially within a dilution range of 1/2 to 1/20, for example 1/2 to 1/4; in particular, the plasma is diluted by 1/3.

5. A method as claimed in any one of claims 1 to 4, **characterized in that** the fibrin polymerization inhibitor is a polypeptide inhibitor, for example H-GlyProArgPro-OH.AcOH (GPRP.AcOH) or is an anti-fibrinogen antibody.

6. A method as claimed in any one of claims 1 to 5, **characterized in that** the inhibitor of the activity of TFPI on the circulating tissue factor is constituted by anti-TFPI antibody or functional antibody fragments binding the TFPI that are capable of inhibiting the activity of TFPI on circulating tissue factor, for example a monoclonal T4E2 antibody or a functional fragment of said antibody.

7. A method as claimed in any one of claims 1 to 6, comprising the steps of:
a) bringing a plasma sample into contact with a reagent 1 comprising a fibrin polymerization inhibitor, an inhibitor of the activity of TFPI on cTF and, if appropriate, a dilution buffer and/or a heparin inhibitor, for example polybrene;
b) incubating the reaction medium constituted in step a) with a reagent 2 comprising factor X in excess, factor VII in excess and calcium ions in solution, in order to produce activated factor X (FXa);
c) after incubation, bringing the reaction medium constituted in step b) into contact with an enzymatic substrate for activated factor X (FXa);
d) detecting the transformation of the factor Xa substrate.

8. A method as claimed in claim 7, **characterized in that** the plasma sample is diluted by 1/3, in an Owren Koller buffer supplemented with 1.2 mg/L polybrene and 50 µg/L of anti-TFPI antibody.

9. A method as claimed in any one of claims 7 or 8, **characterized in that** the quantitative measurement of the transformation of the enzymatic substrate for factor Xa is carried out by reading the optical density in a pre-determined window.

10. A method as claimed in any one of claims 7 to 9, **characterized in that** the quantity of transformed factor Xa substrate is assayed by referring to a calibration curve.

11. A method as claimed in any one of claims 7 to 10, **characterized in that** the reaction is carried out under the following conditions:
• for step a), the plasma sample diluted by 1/3 in an Owren Koller buffer is brought into contact with 50 µg/L of anti-TFPI antibody and 1.2 mg/L of polybrene and a fibrin polymerization inhibitor designated GPRP.AcOH in a concentration of 10 g/L;
• for step b), 25 µl of factor X in a proportion of 80 Plasma Equivalent Unit (PEU)/mg/mL, 25 µl of factor VII in a proportion of 137 Plasma Equivalent Unit (PEU)/mg/mL and 50 µl of a 25 mM CaCl₂ solution are added to 50 µl of the mixture of step a),and the reaction mixture obtained is incubated at 37°C for 500 seconds;
• for step c), 100 µl of a solution of the enzymatic substrate for activated factor X is added and the quantity of transformed enzymatic substrate is determined by optical density reading in a reading window of 6 to 600 s, at a wavelength of 405 nm.

12. A method as claimed in any one of claims 1 to 11, **characterized in that** it further comprises assaying an internal control.

13. A method as claimed in any one of claims 1 to 12, **characterized in that** the optical density values obtained are compared with those of a calibration curve prepared using a pool of normal plasma enriched in tissue factor or using a NaCl-CaCl₂ solution enriched in tissue factor.

14. A kit for measuring the activity of circulating tissue factor in a biological medium in a biological sample, in particular in a plasma sample, comprising:
• a reagent 1 comprising a dilution buffer for the tested sample, a fibrin polymerization inhibitor, a TFPI inhibitor and, if appropriate, a heparin inhibitor;
• purified or recombinant factor VII, advantageously lyophilized;
• purified or recombinant factor X, advantageously lyophilized;
• calcium ions, for example in the form of CaCl₂;
• an enzymatic substrate for activated factor X, advantageously lyophilized;
• if appropriate a calibration agent, advantageously lyophilized.

15. A kit as claimed in claim 14, **characterized in that** the sample dilution buffer is an Owren Koller buffer, the heparin inhibitor is polybrene and the fibrin polymerization inhibitor is H-GlyProArgPro-OH.AcOH (GPRP.AcOH) or is an anti-fibrinogen antibody.

16. A kit as claimed in claim 14 or claim 15, **characterized in that** it further comprises an internal control and/or a calibration curve for the activity of factor Xa.

17. A method for the *in vitro* detection of a coagulation anomaly, comprising measuring the activity of circulating tissue factor as claimed in any one of claims 1 to 13, and optionally the comparison of the level of the measured circulating tissue factor activity with a normal value for said activity.

18. A method as claimed in claim 17, wherein the normal value is obtained after assaying the circulating tissue factor activity in a pool of normal plasma samples or is established from values for the activity of the circulating tissue factor measured on individually assayed normal plasma samples.

19. A method as claimed in any one of claims 17 to 18 or a kit as claimed in any one of claims 14 to 16, for the detection of an increase in the rate of circulating tissue factor associated with a risk of thrombosis or with a thrombosis, in particular with the symptoms of acute myocardial infarction (AMI), acute coronary syndrome, disseminated intravascular coagulation (DIVC), diabetes, preeclampsia, miscarriage, thrombosis, sepsis, inflammation or cancer.
